(19)

Europäisches Patentamt
European Patent Office
Office européen des brevets

(11) **EP 3 929 936 A1**

(12) **EUROPEAN PATENT APPLICATION**

(43) Date of publication:
**29.12.2021 Bulletin 2021/52**

(21) Application number: **21180032.1**

(22) Date of filing: **17.06.2021**

(51) Int Cl.:
**G16H 30/40** *(2018.01)*     **G16H 50/20** *(2018.01)*
**G16H 50/80** *(2018.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.06.2020 US 202016946435**

(71) Applicant: **Siemens Healthcare GmbH**
**91052 Erlangen (DE)**

(72) Inventors:
 • **CHAGANTI, Shikha**
 **Princeton, 05480 (US)**
 • **GRBIC, Sasa**
 **Plainsboro, 08536 (US)**
 • **GEORGESCU, Bogdan**
 **Princeton, 08540 (US)**
 • **CHABIN, Guillaume**
 **75012 Paris (FR)**
 • **RE, Thomas**
 **Monroe, 08831 (US)**
 • **YOO, Youngjin**
 **Princeton, 08540 (US)**
 • **FLOHR, Thomas**
 **91486 Uehlfeld (DE)**
 • **ZIEBANDT, Valentin**
 **90482 Nürnberg (DE)**
 • **COMANICIU, Dorin**
 **Princeton, 08540 (US)**

(74) Representative: **Horn Kleimann Waitzhofer**
**Patentanwälte PartG mbB**
**Ganghoferstrasse 29a**
**80339 München (DE)**

(54) **AUTOMATIC DETECTION OF COVID-19 IN CHEST CT IMAGES**

(57)     Systems and methods for automatically detecting a disease in medical images are provided. Input medical images are received. A plurality of metrics for a disease is computed for each of the input medical images. The input medical images are clustered into a plurality of clusters based on one or more of the plurality of metrics to classify the input medical images. The plurality of clusters comprise a cluster of one or more of the input medical images associated with the disease and one or more clusters of one or more of the input medical images not associated with the disease. In one embodiment, the disease is COVID-19 (coronavirus disease 2019).

100

Receive input medical images
102

Compute a plurality of metrics for a disease for each of the input medical images
104

Cluster the input medical images into a plurality of clusters based on one or more of the plurality of metrics to classify the input medical images
106

Output the classification of the input medical images
108

Figure 1

**Description**

CROSS-REFERENCE TO RELATED APPLICATIONS

Certain embodiments described herein may be related to U.S. Patent Application No. 16/837,979, filed April 1, 2020, the disclosure of which is incorporated herein by reference in its entirety.

TECHNICAL FIELD

The present invention relates generally to automatic detection of COVID-19 (coronavirus disease 2019) in chest CT (computed tomography) images, and in particular to automatic classification of chest CT images to distinguish COVID-19 from other pulmonary diseases using machine learning.

BACKGROUND

COVID-19 (coronavirus disease 2019) is an infectious disease caused by the severe-acute respiratory symptom coronavirus 2 (SARS-Cov2). COVID-19 presents such respiratory symptoms as coughing, difficulty breathing, pneumonia, and SARS (severe acute respiratory syndrome). In the current clinical practice, COVID-19 is diagnosed via RT-PCR (reverse transcription polymerase chain reaction).

Typically, a patient suspect of, or confirmed as, having COVID-19 receives CT imaging of the chest to evaluate the lungs of the patient. Recently, techniques have been proposed for detecting COVID-19 in CT images. However, it is not clear whether conventional techniques are able to distinguish CT images of COVID-19 not only from CT images of healthy patients, but also from CT images of other pulmonary disease, such as other infections, malignancy, ILD (interstitial lung disease), and COPD (chronic obstructive pulmonary disease). This is especially important as COVID-19 can manifest similarly to other pulmonary diseases, which can lead to confusion in triage and diagnosis. In addition, some conventional techniques have been developed with limited generalizability, while other conventional techniques do not provide details, such as acquisition protocols or geographic location of origin, on the imaging data from which the techniques were developed.

BRIEF SUMMARY OF THE INVENTION

In accordance with one or more embodiments, systems and methods for automatically detecting a disease in medical images are provided. Input medical images are received. A plurality of metrics for a disease is computed for each of the input medical images. The input medical images are clustered into a plurality of clusters based on one or more of the plurality of metrics to classify the input medical images. The plurality of clusters comprise a cluster of one or more of the input medical images associated with the disease and one or more clusters of one or more of the input medical images not associated with the disease. In one embodiment, the disease is COVID-19 (coronavirus disease 2019).

In one embodiment, the input medical images are clustered by performing unsupervised hierarchical clustering based on a distance between each pair of images in the input medical images. The distance between each pair of images in the input medical images is computed by computing an initial distance between same metrics of the one or more of the plurality of metrics for each respective pair of images and averaging the initial distances between the same metrics for each respective pair of images.

In one embodiment, the input medical images are clustered by performing a supervised classification using a random forest classifier and a logistic regression classifier.

In one embodiment, the one or more of the plurality of metrics are selected that most discriminate medical images associated with the disease from medical images not associated with the disease. The plurality of metrics for the disease represent the distribution, location, and extent of the disease.

In accordance with one or more embodiments, systems and methods for automatically detecting a disease in medical images are provided. An input medical image of lungs of a patient is received. The lungs are segmented from the input medical image. A probability map for abnormality patterns associated with a disease is generated from the input medical image. A classification of the input medical image is determined based on the segmented lungs and the probability map. The classification represents whether the input medical image is associated with the disease.

In one embodiment, the disease is COVID-19 and the abnormality patterns associated with COVID-19 comprise opacities of one or more of ground glass opacities (GGO), consolidation, and crazy-paving pattern.

In one embodiment, the classification of the input medical image is an indication that the input medical image is associated with the disease or an indication that the input medical image is not associated with the disease.

It is understood that computing metrics may include the step/s of segmenting as disclosed above or below with respect to the embodiments. Clustering may include the step/s of determining a classification or vice versa as disclosed above or below with respect to the embodiments.

These and other advantages of the invention will be apparent to those of ordinary skill in the art by reference to the following detailed description and the accompanying drawings.

BRIEF DESCRIPTION OF THE DRAWINGS

Figure 1 shows a method of a metrics-based approach for classifying medical images, in accordance with one or more embodiments;

Figure 2A shows images depicting the periphery regions of the lungs, in accordance with one or more embodiments;

Figure 2B shows images depicting the rind of the lungs and the core of the lungs, in accordance with one or more embodiments;

Figure 3 shows a framework for classifying a disease in a medical image, in accordance with one or more embodiments;

Figure 4 shows a method for classifying a disease in a medical image, in accordance with one or more embodiments;

Figure 5 shows a table showing division of a dataset for training, validation, and testing, in accordance with one or more embodiments;

Figure 6 shows heatmaps of hierarchical clustering generated according to the metrics-based approach, in accordance with one or more embodiments;

Figure 7 shows a graph comparing the TPR (true positive rate) against the FPR (false positive rate) for the classifiers utilized for the metrics-based approach and the deep learning-based approach, in accordance with one or more embodiments;

Figure 8 shows confusion matrices for classifiers utilized for the metrics-based approach and the deep learning-based approach, in accordance with one or more embodiments;

Figure 9 shows an exemplary artificial neural network that may be used to implement one or more embodiments described herein;

Figure 10 shows a convolutional neural network that may be used to implement one or more embodiments described herein; and

Figure 11 shows a high-level block diagram of a computer that may be used to implement one or more embodiments described herein.

DETAILED DESCRIPTION

The present invention generally relates to methods and systems for automatic detection of COVID-19 (coronavirus disease 2019) in chest CT (computed tomography) images. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

COVID-19 is an infectious disease that typically presents such respiratory symptoms as fever, cough, and difficulty breathing. Typically, patients suspected of, or confirmed as, having COVID-19 receive CT imaging of the chest in order to assess the lungs of the patient. For patients with COVID-19, such CT imaging depicts abnormality patterns associated with COVID-19. However, other pulmonary diseases, such as, e.g., other infections (e.g., influenza), malignancy, ILD (interstitial lung disease), and COPD (chronic obstructive pulmonary disease), similarly manifest in the lungs of the patient, and thus such CT imaging of patients with other pulmonary diseases may depict similar abnormality patterns.

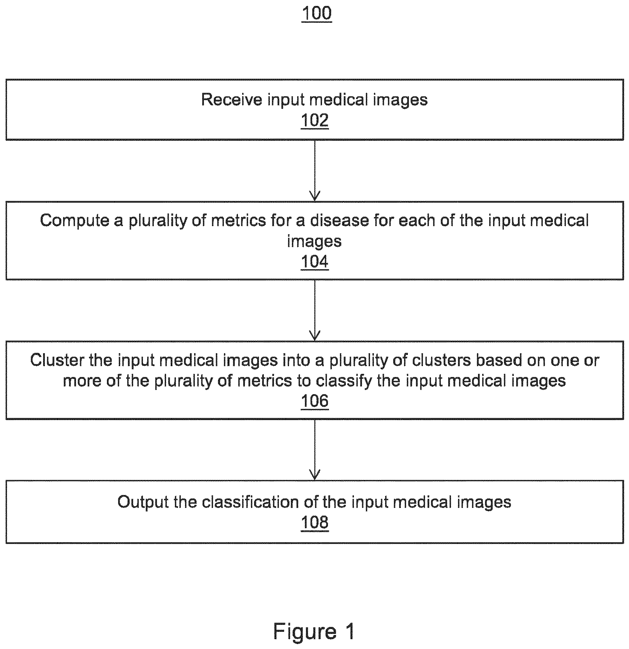

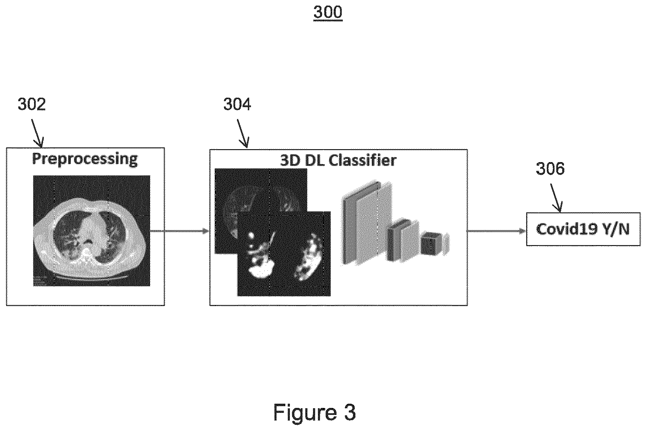

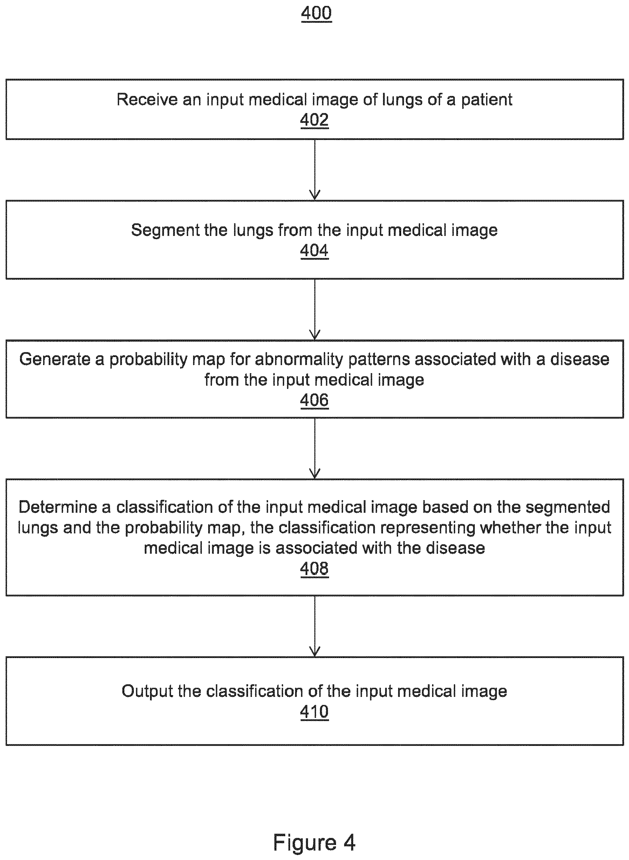

Embodiments described herein distinguish CT images of abnormality patterns associated with COVID-19 from CT images of abnormality patterns associated with other pulmonary diseases and from CT images of patterns associated with healthy tissue to provide for automatic detection of COVID-19 in CT images. In one embodiment, a metrics-based approach is performed to automatically detect COVID-19 in CT images, as described with respect to, e.g., Figures 1 and 4. In another embodiment, a deep learning-based approach is performed to automatically detect COVID-19 in CT images, as described with respect to, e.g., Figures 3-4. Advantageously, the automatic detection of COVID-19 in CT images, in accordance with embodiments described herein, may be utilized to augment radiologist diagnostic accuracy and efficiency.

It should be understood that while embodiments described herein are described with respect to detection of COVID-19 in medical images, such embodiments are not so limited. Embodiments may be applied for the detection of any disease, such as, e.g., other types of viral pneumonia (e.g., SARS (severe acute respiratory syndrome), MERS (Middle East respiratory syndrome), etc.), bacterial pneumonia, fungal pneumonia, mycoplasma pneumonia, and other types of pneumonia and other types of diseases (e.g., ILD, COPD). Further, as used herein, COVID-19 includes mutations of the COVID-19 virus (which may be referred to by different terms).

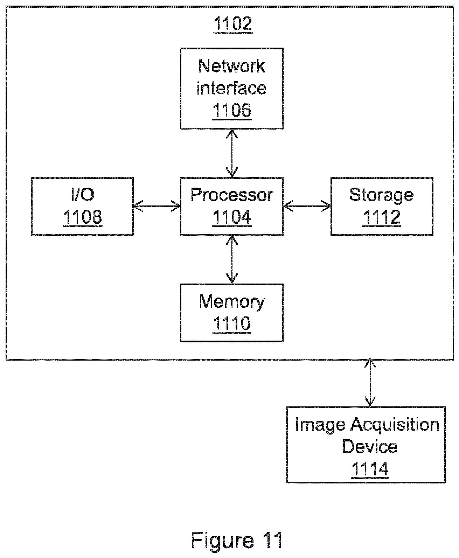

Figure 1 shows a method 100 of a metrics-based approach for classifying medical images, in accordance with one or more embodiments. Method 100 may be performed by one or more suitable computing devices, such as, e.g., computer 1102 of Figure 11.

At step 102, input medical images are received. In one embodiment, the input medical images comprise images of lungs of patients with a disease and/or without a disease (i.e., healthy tissue). The disease may include COVID-19, pneumonia, ILD, COPD, etc. Accordingly, the input medical images may comprise images depicting abnormality patterns associated with the disease. For example, where the disease is COVID-19, the input medical images may show opacities such as, e.g., GGO (ground glass opacity), consolidation, crazy-paving pattern, atelectasis, interlobular septal thickening, pleural effusions, bronchiectasis, etc.

In one embodiment, the input medical images are CT input medical images. However, the input medical images may be of any suitable modality, such as, e.g., MRI (magnetic resonance imaging), US (ultrasound), x-ray, or any other modality or combination of modalities. The input medical images may comprise 2D images or 3D volumes, and each input medical image may be a single image (or volume) or a plurality of images (e.g., a time sequence of images). The input medical images may be received directly from an image acquisition device, such as, e.g., a CT scanner, as the input medical images are acquired, or can be received by loading previously acquired input medical images from a storage or memory of a computer system or receiving the input medical images from a remote computer system.

At step 104, a plurality of metrics for a disease are computed for each of the input medical images. In one embodiment, the disease is COVID-19, but the disease may be any other disease (e.g., pneumonia, ILD, COPD, or other lung diseases). In one embodiment, the metrics are computed by first segmenting the lungs and lobes of the lungs from the input medical images. Abnormality patterns associated with the disease are then identified, e.g., using a DenseUNet. Based on the segmented lungs and lobes and the identified abnormality patterns, the metrics for the disease are computed. The metrics represent the severity (e.g., the distribution, location, and extent) of the disease in the lungs.

In one embodiment, the lungs and lobes are segmented from the input medical images by first detecting anatomical landmarks throughout the input medical images using multi-scale deep reinforcement learning. Regions of interest (ROI) of the input medical images are then extracted based on the detected landmarks. Specifically, the lung ROI is extracted using the detected landmark of the carina bifurcation. Other detected landmarks may additionally or alternatively be utilized. For example, the sternum tip may be used to extract the lung ROI from the input medical images where the carina bifurcation is beyond the image field of view of the input medical images. The size and the relative location of the lung ROI towards the carina bifurcation (or other detected landmark) are specified according to annotated data. Next, the extracted lung ROI image is resampled to, e.g., a 2mm isotropic volume and fed into a trained deep image-to-image network (DI2IN) to generate a segmentation mask within the lung ROI. Finally, the segmentation mask is transferred to a unique mask having the same dimension and resolution as the input medical image. The unique mask is output as the final lung segmentation mask. The lobes may be similarly segmented. The DI2IN is trained during a prior offline or training stage. In one embodiment, the DI2IN is trained on a cohort of patients without the prevalence of viral pneumonia and fine-tuned on another cohort with abnormality regions including consolidation, effusions, masses, etc. to improve the robustness of the lung segmentation over the infected area.

In one embodiment, for example where the disease is COVID-19, thirty metrics for COVID-19 are computed. The thirty metrics are as follows:

- Metrics 1-6: Percent of Opacity (PO) computed as the total percent volume of the lung parenchyma affected by the disease for each of the five lobes of the lungs and for the lungs as a whole.

- Metrics 7-12: Percent of High Opacity (PHO) computed as the total percent volume of the lung parenchyma that is severely affected by the disease for each of the five lobes of the lungs and for the lungs as a whole. Regions of the lung parenchyma that are severely affected may be high opacity regions (e.g., abnormality pattern regions with a mean HU (Hounsfield units) greater than -200, corresponding to consolidation and vascular thickening).

- Metrics 13-18: Percentage of High Opacity 2 (PHO2) computed as the total percent volume of the lung parenchyma affected by denser airspace disease for each of the five lobes of the lungs and for the lungs as a whole. Regions of the lung parenchyma affected by denser airspace disease may be high opacity regions (e.g., abnormality pattern regions with a mean HU between -200 and 50, corresponding to consolidation).

- Metric 19: Lung Severity Score (LSS) computed as the sum of a severity score of each of the five lobes of the lungs. In one embodiment, the severity score for each lobe is based on the PO for each lobe. For example, a severity score of a lobe may be: 0 if a lobe is not affected by the disease, 1 if the lobe has 1-25% PO, 2 if the lobe has 26-50% PO, 3 if the lobe has 51-75% PO, and 4 if the lobe has 76-100% PO. The severity score for computing LSS may be based on any other suitable metric.

- Metric 20: Lung High Opacity Score (LHOS) computed as the sum of a severity score of each of the five lobes of the lungs for high opacity regions only. In one embodiment, the severity score for each lobe is based on the PHO for each lobe. For example, a severity score of a lobe may be: 0 if a lobe is not affected by the disease, 1 if the lobe has 1-25% PHO, 2 if the lobe has 26-50% PHO, 3 if the lobe has 51-75% PHO, and 4 if the lobe has 76-100% PHO. The severity score for computing LHOS may be based on any other suitable metric.

- Metric 21:Lung High Opacity Score 2 (LHOS2) computed as the sum of a severity score for each of the five lobes of the lungs for high opacity regions excluding vasculature. Vasculature may be identified based on threshold (e.g., regions with a HU above 50 may be exlucded. In one embodiment, the severity score for each lobe is based on the PHO for each lobe. For example, a severity score of a lobe may be: 0 if a lobe is not affected by the disease, 1 if the lobe has 1-25% PHO, 2 if the lobe has 26-50% PHO, 3 if the lobe has 51-75% PHO, and 4 if the lobe has 76-100% PHO. The severity score for computing LHOS2 may be based on any other suitable metric.

- Metric 22: Bilaterality determined as true if both lungs are affected by the disease and false if only one or none of the lungs are affected by the disease.

- Metric 23: Number of lobes affected by the disease.

- Metric 24: Number of total lesions in the lungs.

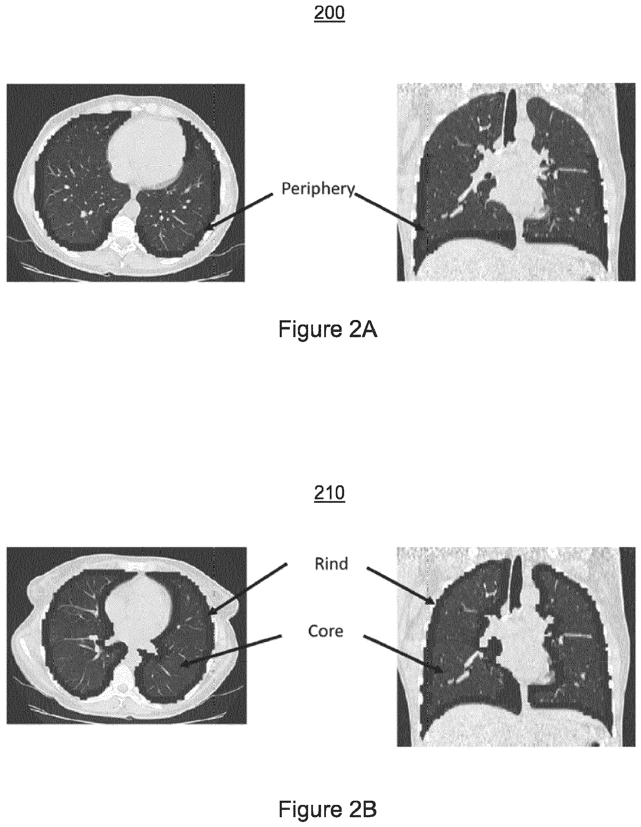

- Metric 25: Number of peripheral lesions determined as the number of lesions that are in the periphery of the lungs (which excludes the apex and mediastinal regions). Figure 2A shows images 200 depicting the periphery regions of the lungs, in accordance with one or more embodiments.

- Metric 26: Number of lesions in the rind of the lungs. Any abnormality that intersects with the rind is considered a lesion in the rind. Figure 2B shows images 210 depicting the rind of the lungs, in accordance with one or more embodiments.

- Metric 27: Number of lesions in the core of the lungs. Any abnormality that does not intersect with the rind is considered a lesion in the core. Images 210 in Figure 2B shows the core of the lungs.

- Metric 28: Percent of peripheral distribution computed as the number of peripheral lesions divided by the number of total lesions.

- Metric 29: Percent of peripheral lesions computed as the total percent volume of the lung parenchyma affected by the disease for peripheral lesions only.

- Metric 30: Percent of GGO computed as the total percent volume of the lung parenchyma affected by less dense airspace disease (i.e., lesions characterized as GGO only). GGO is the abnormality pattern regions with a mean HU less than -200.

At step 106, the input medical images are clustered into a plurality of clusters based on one or more of the plurality of metrics to classify the input medical images. The plurality of clusters comprise a cluster of one or more of the input medical images that are associated with the disease and one or more clusters of one or more input medical images that are not associated with the disease (i.e., associated with other diseases or associated with healthy tissue).

In one embodiment, the one or more of the plurality of metrics are selected, from the plurality of metrics, as the metrics that most discriminate between abnormality patterns associated with the disease and patterns not associated with the disease (i.e., abnormality patterns associated with other diseases or patterns associated with healthy tissue). The one or more of the plurality of metrics may be selected using mutual information based on an internal validation split.

In one embodiment, the input medical images are clustered based on the one or more of the plurality of the selected metrics using unsupervised hierarchical cluster analysis to cluster input medical images that have similar features. A distance matrix is computed by calculating, for each pair of the input medical images, an initial distance between same metrics of the one or more of the plurality of metrics. For example, the initial distance between the PO metric is calculated for each pair of input medical images or the initial distance between the PHO metric is calculated for each pair of input medical images. The initial distance may be any suitable distance measure, such as, e.g., the pairwise Euclidean distance. Average linkage clustering is then used to hierarchically cluster the input medical images using the average of the initial distances between the same metrics for each pair of input medical images.

In one embodiment, the input medical images are clustered based on the one or more of the plurality of metrics using supervised classification. Two metrics-based classifiers are trained. First, a random forest classifier is trained using the one or more of the plurality of metrics. Subsequently, a logistic regression classifier is trained after a feature transformation based on gradient boosted trees on all of the plurality of metrics. The random forest classifier and the logistic regression classifier are trained during a prior offline or training stage. Once trained, the random forest classifier and the logistic regression classifier are applied at step 106 during an online or inference stage. For instance, the plurality of selected metrics are computed and the random forest classifier and the logistic regression classifier are applied to provide a class score, which is used to classify the images. In one embodiment, the gradient boosted trees were trained using 2000 estimators with a max depth of 3 and 3 features for each split. A boosting fraction of 0.8 was used for fitting the individual trees. The LR classifier was trained with L2 regularization (C=0.1). Class weights were adjusted to class frequencies to address class imbalance between disease cases and non-disease cases.

At step 108, the classification of the input medical images is output. For example, the classification of the input medical images can be output by displaying the classification of the input medical images on a display device of a computer system, storing the classification of the input medical images on a memory or storage of a computer system, or by transmitting the classification of the input medical images to a remote computer system.

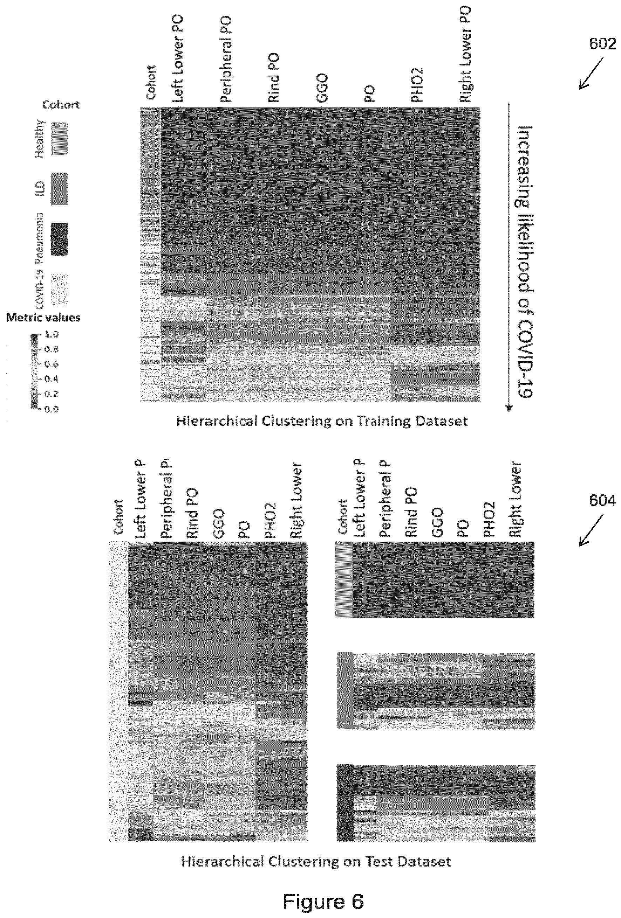

In one embodiment, the classification of the input medical images may be output as a heatmap. Exemplary heatmaps are shown in Figure 6, which is described in more detail below.

Figure 3 shows a framework 300 for classifying a disease in a medical image, in accordance with one or more embodiments. Figure 4 shows a method 400 for classifying a disease in a medical image, in accordance with one or more embodiments. Figures 3 and 4 will be described together. The steps of Figure 4 may be performed by one or more suitable computing devices, such as, e.g., computer 1102 of Figure 11.

At step 402, an input medical image of lungs of a patient is received. In one embodiment, the input medical image is a CT medical image. However, the input medical image may be of any suitable modality, such as, e.g., MRI, US, x-ray, or any other modality or combination of modalities. The input medical image may comprise a 2D image or 3D volume, and may be a single image or a plurality of images (e.g., a time sequence of images). The input medical image may be received directly from an image acquisition device, such as, e.g., a CT scanner, as the input medical image is acquired, or can be received by loading a previously acquired input medical image from a storage or memory of a computer system or receiving an input medical image from a remote computer system.

At step 404, the lungs are segmented from the input medical image. In one example, the lungs are segmented at preprocessing step 302 of Figure 3. The lungs may be segmented from the input medical image as described above with respect to step 104 of Figure 1.

At step 406, a probability map for abnormality patterns associated with a disease is generated from the input medical image. In one example, the probability map is generated at preprocessing step 302 of Figure 3. In one embodiment, the disease is COVID-19 and the abnormality regions associated with COVID-19 include opacities such as, e.g., GGO, consolidation, crazy-paving pattern, atelectasis, interlobular septal thickening, pleural effusions, bronchiectasis, etc. However, the disease may be any other disease (e.g., pneumonia, ILD, COPD, or other lung diseases).

The probability map for abnormality patterns associated with the disease may be generated using a machine learning based opacity classifier, such as, e.g., a DenseUNet. However, any other suitable machine learning based network may be applied for generating a probability map. The DenseUNet with anisotropic kernels is trained to transfer the input medical image to a probability map of the same size. All voxels in the lungs that fully or partially comprise GGO, consolidations, or crazy-paving patterns (or any other type of abnormality associated with the disease) are defined as positive voxels. The remainder of the image area within the lungs and the entire area outside the lungs are defined as negative voxels. The DenseUNet is trained in an end-to-end system. An initial probability mask generated by the DenseUNet is filtered using the segmented lungs so that only the abnormality regions present within the lungs are identified.

The filtered probability mask is output as a final probability map for abnormality patterns associated with the disease. The final probability map may be overlaid on the input medical image. In one embodiment, the probability map may be converted to a binary segmentation mask based on a threshold (e.g., 0.5).

At step 408, a classification of the input medical image is determined based on the segmented lung and the probability map. The classification represents whether the input medical image is associated with the disease. In one example, the classification is a score between 0 and 1. The classification may be a binary classification (e.g., yes or no) that the input medical image is associated with the disease or that the input medical image is not associated with the disease based on the score using a threshold.

In one embodiment, the classification of the input medical image is determined using a machine learning based classifier. For example, the classifier may be 3D deep learning classifier 304 in Figure 3. The classifier receives as input the input medical image masked by the segmented lung and the probability map. In one embodiment, the classifier uses anisotropic 3D kernels to balance resolution and speed, and comprises deep dense blocks that gradually aggregate features down to a binary output. The classifier may be trained during a prior offline or training stage in an end-to-end manner as a classification system using binary cross entropy and uses probabilistic sampling of the training data to adjust for the imbalance in the training dataset labels. Once trained, the classifier is applied at step 408 during an online or inference stage.

At step 410, the classification of the input medical image is output. In one example, the classification of the input medical image is output as output 306 of Figure 3 representing a yes or no indication that either the input medical image is associated with the disease or that the input medical image is not associated with the disease. The classification of the input medical image can be output by displaying the classification of the input medical image on a display device of a computer system, storing the classification of the input medical image on a memory or storage of a computer system, or by transmitting the classification of the input medical image to a remote computer system.

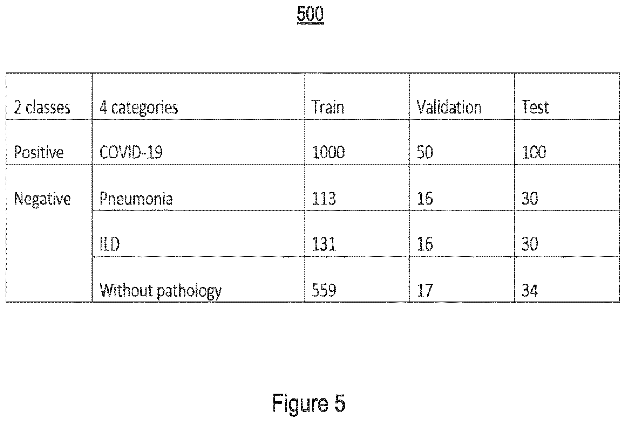

The metrics-based approach (as described with respect to, e.g., Figure 1) and the deep learning-based approached (as described with respect to, e.g., Figure 4), in accordance with embodiments described herein, were experimentally validated for detecting COVID-19 using a dataset of 2,096 CT images, which included 1150 CT images of patients with COVID-19 and 946 CT images of patients without COVID-19. The 946 CT images of patients without COVID-19 included 159 CT images of patients with pneumonia, 177 CT images of patients with ILD, and 610 CT images without any lung disease. The CT images were acquired from 16 different clinical centers in North America and Europe. The CT images of patients with COVID-19 acquired from North America were confirmed via RT-PCR testing, while the CT images of patients with COVID-19 acquired from Europe 19 were confirmed by either RT-PCR testing or diagnosed by a clinician based on clinical symptoms, epidemiological exposure, and radiological assessment. The pneumonia cohort comprised cases of patients with non-COVID-19 viral pneumonias, organizing pneumonia, or aspiration pneumonia. The ILD cohort comprised patients with various types of ILD exhibiting GGO, reticulation, honeycombing, and consolidation to different degrees. The dataset was divided into training, validation, and testing datasets. Model training and selection was performed based on the training and validation sets. Figure 5 shows a table 500 showing division of the dataset for training, validation, and testing.

The metrics-based approach was implemented using a deep image-to-image network trained on a large cohort of healthy and abnormal cases for segmentation of the lungs and lobes of the lungs. A DenseUNet was used to identify abnormality patterns associated with COVID-19. Thirty metrics (as described above with respect to step 104 of Figure 1) were computed representing the severity of COVID-19. Seven metrics that were most discriminative between COVID-19 and non-COVID-19 patterns were selected by comparing mutual information between the metrics and the class in the training dataset of 999 COVID-19 cases and 801 controls cases (pneumonia, ILD, and healthy). One COVID-19 case was excluded from training due to field of view issues, one pneumonia control case was excluded since the z-axis resolution was less than 10 mm, and another pneumonia control case was excluded due to incorrect DICOM (digital imaging and communications in medicine) parameters and artifact issues.

The selected metrics were percent of GGO, PHO2 (corresponding to consolidation), PO (corresponding to consolidation and GGO), percent of opacities in the periphery, percent of opacities in the rind, percent of opacities in the right lower lobe, and percent of opacities in the left lower lobe. The selected metrics correspond to typical COVID-19 characteristics (i.e., multifocal GGO and consolidation with basilar and peripheral distribution of the disease) reported in clinical literature.

Figure 6 shows heatmaps of hierarchical clustering generated according to the metrics-based approach, in accordance with one or more embodiments. Heatmap 602 shows hierarchical clustering on the training dataset and heatmap 604 shows hierarchical clustering on the test dataset. The ground truth diagnosis cohort membership (COVID-19, pneumonia, ILD, and healthy) is shown by shading (or color). The metric values are standardized and rescaled to a value between 0 and 1. The probability of belonging to the COVID-19 class increases towards the bottom of each heatmap 602 and 604, which corresponds to higher values of the metrics (i.e., more opacities (both GGO and consolidation) and more peripheral and basilar distribution). In heatmap 602, the clustering is performed on the entire training set of 1800 patients. The middle of heatmap 602 shows an ambiguous region, where there is an overlap of features from different disease cohorts. Heatmap 604 shows the same clustering in the test dataset for each of the disease cohorts. While there is a cluster of COVID-19 subjects that have characteristic features, there are also many which do not show all characteristics. Moreover, some cases of pneumonia and ILD overlap with typical features of COVID-19.

The deep learning-based approach was implemented using a deep learning-based 3D neural network model trained to separate the positive class (COVID-19 class) from the negative class (non-COVID-19 class). A two-channel 3D tensor, with a first channel comprising the CT image masked by the lung segmentation and a second channel comprising a probability map of abnormality patterns associated with COVID-19. The 3D network used anisotropic 3D kernels to balance resolution and speed, and was formed of deep dense blocks that gradually aggregate features down to a binary output. The network was trained in an end-to-end manner as a classification system using binary cross entropy and probabilistic sampling of the training data to adjust for the imbalance in the training dataset labels. A separate validation dataset was used for final model selection before performance was measured on the testing set. The input 3D tensor size was fixed (2 x 128 x 384 x 384) corresponding to the lung segmentation from the CT image rescaled to a 3 x 1 x 1 mm resolution. The first two blocks were anisotropic comprising convolution (kernels 1 x 3 x 3) - batch normalization - LeakyReLU (leaky rectified linear unit) and max-pooling (kernels 1 x 2 x 2, stride 1 x 2 x 2). The subsequent five blocks were isotropic with convolution (kernels 3 x 3 x 3) - batch normalization - LeakyReLU and max-pooling (kernels 2 x 2 x 2, stride 2 x 2 x 2) followed by a final linear classifier with the input 144-dimensional.

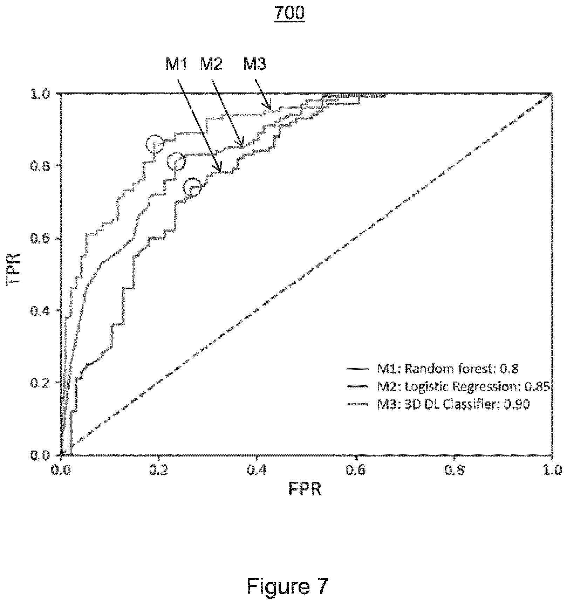

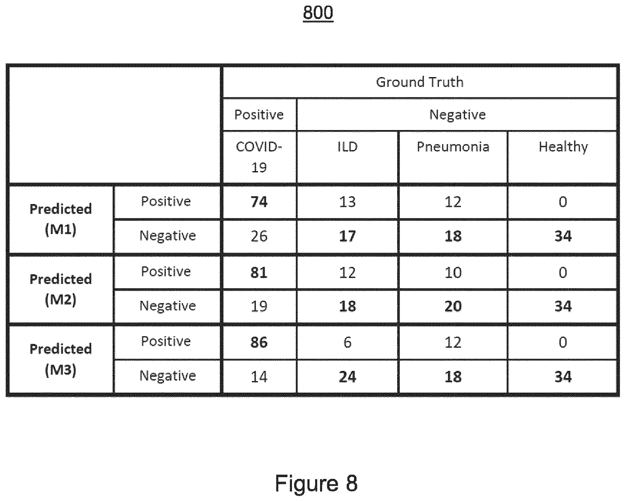

Figure 7 shows a graph 700 comparing the TPR (true positive rate) against the FPR (false positive rate) for the classifiers utilized for the metrics-based approach and the deep learning-based approach, in accordance with one or more embodiments. The dashed diagonal line in graph 700 corresponds to random chance. The random forest classifier, denoted M1, was trained for the metrics-based approach using the seven selected metrics. As shown in graph 700, the performance of the random forest classifier on the test dataset had an AUC (area under curve) of 0.80. The red circles denote the optimal operating point, which yielded a sensitivity of 0.74 and a specificity of 0.73 for the random forest classifier. The performance of the random forest classifier was improved by training a logistic regression classifier, denoted M2, on all thirty metrics. The metrics were first transformed to a higher-dimensional space using feature embedding with gradient boosted trees. The logistic regression classifier produces an AUC of 0.85 with a sensitivity of 0.81 and a specificity of 0.77. While the performance of the logistic regression classifier improved over the random forest classifier, some of the interpretability was lost since the features were transformed to a higher dimension. The deep learning based classifier, denoted M3, had the best performance with an AUC of 0.90, improving the sensitivity and specificity of the system to 0.86 and 0.81 respectively. The improvement is mostly due to the reduction of the false positives from the ILD and non-COVID-19 pneumonia categories. The optimal operating point, circled in graph 700, for all models was chosen as the point on the ROC (receiver operating characteristic) curve with the shortest distance from the top left corner of graph 700. The corresponding confusion matrices for all three classifiers is shown in table 800 of Figure 8.

The unsupervised clustering on the selected metrics showed that while there are dominant characteristics that can be observed in COVID-19, such as the presence of GGO as well as peripheral and basal distribution, these characteristics are not observed in all cases of COVID-19. On the other hand, some subjects with ILD and pneumonia can exhibit similar characteristics. It was found that the performance of the unsupervised clustering approach can be improved by mapping the metrics into a higher dimensional space prior to training, as shown by the logistic regression classifier in Figure 7. The best classification accuracy was achieved by the deep learning based approach, which may be represented as a high-dimensional, non-linear model.

The deep learning approach achieved a reduced false positive and false negative rate relative to the metrics-based classifier, suggesting that there might be other latent radiological representations of COVID-19 that distinguish it from interstitial lung diseases or other types of pneumonia. The proposed deep learning approach was trained and tested on a dataset of 2096 CT images with 1150 COVID-19 patients and 946 images coming from other categories. The proposed deep learning approach was compared to conventional methods and it was found that the proposed deep learning approach achieved a higher AUC as well as sensitivity.

The experimental validation was performed using a diverse dataset of CT images, which were acquired from a variety of manufacturers, institutions, and regions, ensuring that the results are robust and likely generalizable to different environments. Included in the COVID-19 negative class were not only healthy subjects, but also various types of lung pathology (e.g., ILD and pneumonia).

Embodiments described herein provide clinical value in several aspects. Embodiments described herein may be used for rapid triage of positive cases, particularly in resource constrained environments where radiologic expertise may not be immediately available and RT-PCR results may take up to several hours. Embodiments described herein may help radiologists to prioritize interpreting CT images in patients with COVID-19 by screening out lower probability cases. In addition to rapidity and efficiency concerns, the output of the deep learning approach is easily reproducible and replicable, mitigating inter-reader variability in manually read radiology studies. While RT-PCR is the standard for confirmatory diagnosis of COVID-19, machine learning methods applied to quantitative CT can be performed for diagnosis of COVID-19 with high diagnostic accuracy, increasing the value of imaging in diagnosis and management of COVID-19.

Further, embodiments described herein may be integrated in surveillance of patients for COVID-19, even in unsuspected patients. For example, all chest CT images for pulmonary and non-pulmonary pathology (e.g., coronary artery exams, chest trauma evaluation) may be automatically assessed for evidence of COVID-19 lung disease, as well as for non-COVID-19 pneumonia. Referring clinicians may be alerted for COVID-19 positive determinations, allowing more rapid institution of isolation protocols. Finally, embodiments described herein may be applied retrospectively to large numbers of chest CT images from institutional PACS (picture archiving and communication system) worldwide to uncover the origin and trace the diffuse of SARS-CoV-2 in communities prior to the implementation of widespread testing efforts.

Embodiments described herein may be deployed and validated in a clinical setting to evaluate the clinical utility and diagnostic accuracy on prospective data, as well as to determine the correlation of the various metrics described herein with the clinical severity of COVID-19 and disease progression over time. COVID-19 severity can be further quantified by using features from contrast CT angiography, such as detection and measurement of acute pulmonary embolism, which was reported to be associated with severe COVID-19 infections. In addition, classifiers described herein may be improved by incorporating other clinical data in the training, such as pulse oximetry, cell counts, liver enzymes, etc., in addition to imaging features.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, embodiments described herein are described with respect to methods and systems for automatic detection of COVID-19 in chest CT images using a trained machine learning based network, as well as with respect to methods and systems for training a machine learning based network for automatic detection of COVID-19 in chest CT images. Features, advantages or alternative embodiments herein can be assigned to the other claimed objects and vice versa. In other words, claims for methods and systems for training a machine learning based network can be improved with features described or claimed in context of the methods and systems for utilizing a trained machine learning based network, and vice versa.

In particular, the trained machine learning based network of the methods and systems for automatic detection of COVID-19 in chest CT images can be adapted by the methods and systems for training the machine learning based network for automatic detection of COVID-19 in chest CT images. Furthermore, the input data of the trained machine learning based network can comprise advantageous features and embodiments of the training input data, and vice versa. Furthermore, the output data of the trained machine learning based network can comprise advantageous features and embodiments of the output training data, and vice versa.

In general, a trained machine learning based network mimics cognitive functions that humans associate with other human minds. In particular, by training based on training data, the trained machine learning based network is able to adapt to new circumstances and to detect and extrapolate patterns.

In general, parameters of a machine learning based network can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based network can be adapted iteratively by several steps of training.

In particular, a trained machine learning based network can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

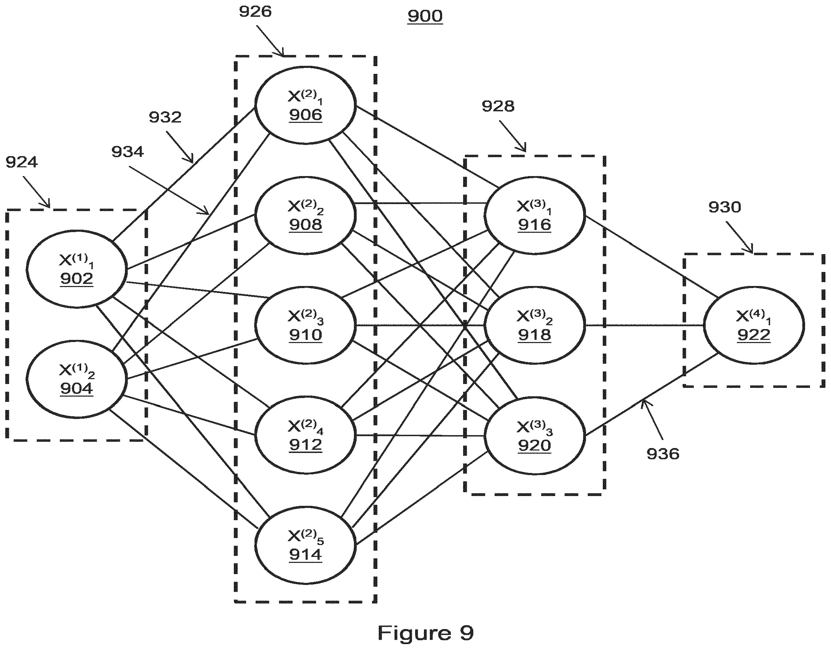

Figure 9 shows an embodiment of an artificial neural network 900, in accordance with one or more embodiments. Alternative terms for "artificial neural network" are "neural network", "artificial neural net" or "neural net". Machine learning networks described herein, such as, e.g., random forest classifier and the logistic regression classifier utilized at step 106 of Figure 1 or the classifier utilized at step 408 of Figure 4, may be implemented using artificial neural network 900.

The artificial neural network 900 comprises nodes 902-922 and edges 932, 934, ..., 936, wherein each edge 932, 934, ..., 936 is a directed connection from a first node 902-922 to a second node 902-922. In general, the first node 902-922 and the second node 902-922 are different nodes 902-922, it is also possible that the first node 902-922 and the second node 902-922 are identical. For example, in Figure 9, the edge 932 is a directed connection from the node 902 to the node 906, and the edge 934 is a directed connection from the node 904 to the node 906. An edge 932, 934, ..., 936 from a first node 902-922 to a second node 902-922 is also denoted as "ingoing edge" for the second node 902-922 and as "outgoing edge" for the first node 902-922.

In this embodiment, the nodes 902-922 of the artificial neural network 900 can be arranged in layers 924-930, wherein the layers can comprise an intrinsic order introduced by the edges 932, 934, ..., 936 between the nodes 902-922.

In particular, edges 932, 934, ..., 936 can exist only between neighboring layers of nodes. In the embodiment shown in Figure 9, there is an input layer 924 comprising only nodes 902 and 904 without an incoming edge, an output layer 930 comprising only node 922 without outgoing edges, and hidden layers 926, 928 in-between the input layer 924 and the output layer 930. In general, the number of hidden layers 926, 928 can be chosen arbitrarily. The number of nodes 902 and 904 within the input layer 924 usually relates to the number of input values of the neural network 900, and the number of nodes 922 within the output layer 930 usually relates to the number of output values of the neural network 900.

In particular, a (real) number can be assigned as a value to every node 902-922 of the neural network 900. Here, $x^{(n)}_i$ denotes the value of the i-th node 902-922 of the n-th layer 924-930. The values of the nodes 902-922 of the input layer 924 are equivalent to the input values of the neural network 900, the value of the node 922 of the output layer 930 is equivalent to the output value of the neural network 900. Furthermore, each edge 932, 934, ..., 936 can comprise a weight being a real number, in particular, the weight is a real number within the interval [-1, 1] or within the interval [0, 1]. Here, $w^{(m,n)}_{i,j}$ denotes the weight of the edge between the i-th node 902-922 of the m-th layer 924-930 and the j-th node 902-922 of the n-th layer 924-930. Furthermore, the abbreviation $w^{(n)}_{i,j}$ is defined for the weight $w^{(n,n+1)}_{i,j}$.

In particular, to calculate the output values of the neural network 900, the input values are propagated through the neural network. In particular, the values of the nodes 902-922 of the (n+1)-th layer 924-930 can be calculated based on the values of the nodes 902-922 of the n-th layer 924-930 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions The transfer function is mainly used for normalization purposes.

In particular, the values are propagated layer-wise through the neural network, wherein values of the input layer 924 are given by the input of the neural network 900, wherein values of the first hidden layer 926 can be calculated based on the values of the input layer 924 of the neural network, wherein values of the second hidden layer 928 can be calculated based in the values of the first hidden layer 926, etc.

In order to set the values $w^{(m,n)}_{i,j}$ for the edges, the neural network 900 has to be trained using training data. In particular, training data comprises training input data and training output data (denoted as $t_i$). For a training step, the neural network 900 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 900 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_k - t^{(n+1)}_j\right) \cdot f\left(\sum_i x^{(n)}_i \cdot w^{(n)}_{i,j}\right)$$

if the (n+1)-th layer is the output layer 930, wherein f is the first derivative of the activation function, and $y^{(n+1)}_j$ is the comparison training value for the j-th node of the output layer 930.

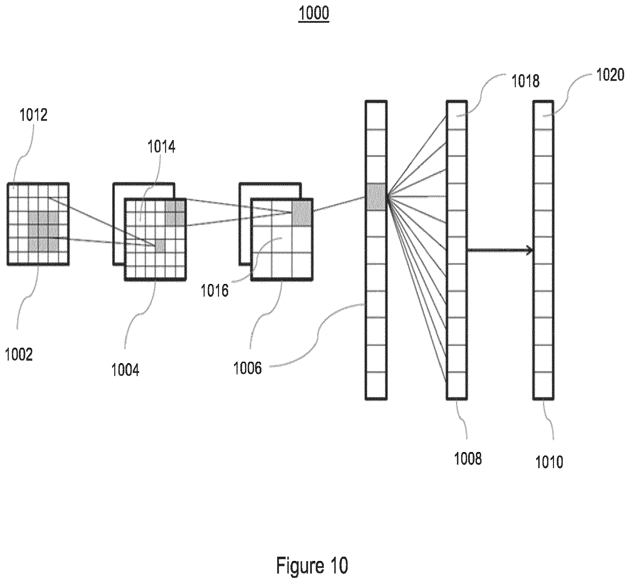

Figure 10 shows a convolutional neural network 1000, in accordance with one or more embodiments. Machine learning networks described herein, such as, e.g., random forest classifier and the logistic regression classifier utilized at step 106 of Figure 1 or the classifier utilized at step 408 of Figure 4, may be implemented using convolutional neural network 1000.

In the embodiment shown in Figure 10, the convolutional neural network comprises 1000 an input layer 1002, a convolutional layer 1004, a pooling layer 1006, a fully connected layer 1008, and an output layer 1010. Alternatively, the convolutional neural network 1000 can comprise several convolutional layers 1004, several pooling layers 1006, and several fully connected layers 1008, as well as other types of layers. The order of the layers can be chosen arbitrarily, usually fully connected layers 1008 are used as the last layers before the output layer 1010.

In particular, within a convolutional neural network 1000, the nodes 1012-1020 of one layer 1002-1010 can be considered to be arranged as a d-dimensional matrix or as a d-dimensional image. In particular, in the two-dimensional case the value of the node 1012-1020 indexed with i and j in the n-th layer 1002-1010 can be denoted as $x^{(n)}{}_{[i,j]}$. However, the arrangement of the nodes 1012-1020 of one layer 1002-1010 does not have an effect on the calculations executed within the convolutional neural network 1000 as such, since these are given solely by the structure and the weights of the edges.

In particular, a convolutional layer 1004 is characterized by the structure and the weights of the incoming edges forming a convolution operation based on a certain number of kernels. In particular, the structure and the weights of the incoming edges are chosen such that the values $x^{(n)}{}_k$ of the nodes 1014 of the convolutional layer 1004 are calculated as a convolution $x^{(n)}{}_k = K_k * x^{(n-1)}$ based on the values $x^{(n-1)}$ of the nodes 1012 of the preceding layer 1002, where the convolution * is defined in the two-dimensional case as

$$x_k^{(n)}[i,j] = \left(K_k * x^{(n-1)}\right)[i,j] = \sum_{i'}\sum_{j'} K_k[i',j'] \cdot x^{(n-1)}[i-i', j-j'] .$$

Here the k-th kernel $K_k$ is a d-dimensional matrix (in this embodiment a two-dimensional matrix), which is usually small compared to the number of nodes 1012-1018 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespective of the number of nodes 1012-420 in the respective layer 1002-1010. In particular, for a convolutional layer 1004, the number of nodes 1014 in the convolutional layer is equivalent to the number of nodes 1012 in the preceding layer 1002 multiplied with the number of kernels.

If the nodes 1012 of the preceding layer 1002 are arranged as a d-dimensional matrix, using a plurality of kernels can be interpreted as adding a further dimension (denoted as "depth" dimension), so that the nodes 1014 of the convolutional layer 1014 are arranged as a (d+1)-dimensional matrix. If the nodes 1012 of the preceding layer 1002 are already arranged as a (d+1)-dimensional matrix comprising a depth dimension, using a plurality of kernels can be interpreted as expanding along the depth dimension, so that the nodes 1014 of the convolutional layer 1004 are arranged also as a (d+1)-dimensional matrix, wherein the size of the (d+1)-dimensional matrix with respect to the depth dimension is by a factor of the number of kernels larger than in the preceding layer 1002.

The advantage of using convolutional layers 1004 is that spatially local correlation of the input data can exploited by enforcing a local connectivity pattern between nodes of adjacent layers, in particular by each node being connected to only a small region of the nodes of the preceding layer.

In embodiment shown in Figure 10, the input layer 1002 comprises 36 nodes 1012, arranged as a two-dimensional 6x6 matrix. The convolutional layer 1004 comprises 72 nodes 1014, arranged as two two-dimensional 6x6 matrices, each of the two matrices being the result of a convolution of the values of the input layer with a kernel. Equivalently, the nodes 1014 of the convolutional layer 1004 can be interpreted as arranges as a three-dimensional 6x6x2 matrix, wherein the last dimension is the depth dimension.

A pooling layer 1006 can be characterized by the structure and the weights of the incoming edges and the activation function of its nodes 1016 forming a pooling operation based on a non-linear pooling function f. For example, in the two dimensional case the values $x^{(n)}$ of the nodes 1016 of the pooling layer 1006 can be calculated based on the values $x^{(n-1)}$ of the nodes 1014 of the preceding layer 1004 as

$$x^{(n)}[i,j] = f(x^{(n-1)}[id_1, jd_2], \dots, x^{(n-1)}[id_1+d_1-1, jd_2+d_2-1])$$

In other words, by using a pooling layer 1006, the number of nodes 1014, 1016 can be reduced, by replacing a number $d1 \cdot d2$ of neighboring nodes 1014 in the preceding layer 1004 with a single node 1016 being calculated as a function of the values of said number of neighboring nodes in the pooling layer. In particular, the pooling function f can be the max-function, the average or the L2-Norm. In particular, for a pooling layer 1006 the weights of the incoming edges are fixed and are not modified by training.

The advantage of using a pooling layer 1006 is that the number of nodes 1014, 1016 and the number of parameters is reduced. This leads to the amount of computation in the network being reduced and to a control of overfitting.

In the embodiment shown in Figure 10, the pooling layer 1006 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 1008 can be characterized by the fact that a majority, in particular, all edges between nodes 1016 of the previous layer 1006 and the nodes 1018 of the fully-connected layer 1008 are present, and wherein the weight of each of the edges can be adjusted individually.

In this embodiment, the nodes 1016 of the preceding layer 1006 of the fully-connected layer 1008 are displayed both as two-dimensional matrices, and additionally as non-related nodes (indicated as a line of nodes, wherein the number of nodes was reduced for a better presentability). In this embodiment, the number of nodes 1018 in the fully connected layer 1008 is equal to the number of nodes 1016 in the preceding layer 1006. Alternatively, the number of nodes 1016, 1018 can differ.

Furthermore, in this embodiment, the values of the nodes 1020 of the output layer 1010 are determined by applying the Softmax function onto the values of the nodes 1018 of the preceding layer 1008. By applying the Softmax function, the sum the values of all nodes 1020 of the output layer 1010 is 1, and all values of all nodes 1020 of the output layer are real numbers between 0 and 1.

A convolutional neural network 1000 can also comprise a ReLU (rectified linear units) layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = max(0,x)$, the tangent hyperbolics function or the sigmoid function.

In particular, convolutional neural networks 1000 can be trained based on the backpropagation algorithm. For preventing overfitting, methods of regularization can be used, e.g. dropout of nodes 1012-1020, stochastic pooling, use of artificial data, weight decay based on the L1 or the L2 norm, or max norm constraints.

In accordance with one embodiment, the neural network used for classification uses anisotropic 3D kernels to balance resolution and speed and consists of deep dense blocks that gradually aggregate features down to a binary output. The network was trained end-to-end as a classification system using binary cross entropy and uses probabilistic sampling of the training data to adjust for the imbalance in the training dataset labels. A separate validation dataset was used for final model selection before the performance was measured on the testing set. The input 3D tensor size is fixed (2 x 128 x 384 x 384) corresponding to the lung segmentation from the CT data rescaled to a 3xlxlmm resolution. The first two blocks are anisotropic and consist of convolution (kernels 1 x 3 x 3) - batch normalization - LeakyReLU and Max-pooling (kernels 1 x 2 x 2, stride 1 x 2 x 2). The subsequent five blocks are isotropic with convolution (kernels 3x3x3) - batch normalization - LeakyReLU and Max-pooling (kernels 2 x 2 x 2, stride 2 x 2 x 2) followed by a final linear classifier with the input 144-dimensional.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 and 4. Certain steps or functions of the methods and workflows described herein, including one or more of the steps or functions of Figures 1 and 4, may be performed by a server or by another processor in a network-based cloud-computing system. Certain steps or functions of the methods and workflows described herein, including one or more of the steps of Figures 1 and 4, may be performed by a client computer in a network-based cloud computing system. The steps or functions of the methods and workflows described herein, including one or more of the steps of Figures 1 and 4, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 and 4, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 1102 that may be used to implement systems, apparatus, and methods described herein is depicted in Figure 11. Computer 1102 includes a processor 1104 operatively coupled to a data storage device 1112 and a memory 1110. Processor 1104 controls the overall operation of computer 1102 by executing computer program instructions that define such operations. The computer program instructions may be stored in data storage device 1112, or other computer readable medium, and loaded into memory 1110 when execution of the computer program instructions is desired. Thus, the method and workflow steps or functions of Figures 1 and 4 can be defined by the computer program instructions stored in memory 1110 and/or data storage device 1112 and controlled by processor 1104 executing the computer program instructions. For example, the computer program instructions can be implemented as computer executable code programmed by one skilled in the art to perform the method and workflow steps or functions of Figures 1 and 4. Accordingly, by executing the computer program instructions, the processor 1104 executes the method and workflow steps or functions of Figures 1 and 4. Computer 1102 may also include one or more network interfaces 1106 for communicating with other devices via a network. Computer 1102 may also include one or more input/output devices 1108 that enable user interaction with computer 1102 (e.g., display, keyboard, mouse, speakers, buttons, etc.).

Processor 1104 may include both general and special purpose microprocessors, and may be the sole processor or one of multiple processors of computer 1102. Processor 1104 may include one or more central processing units (CPUs), for example. Processor 1104, data storage device 1112, and/or memory 1110 may include, be supplemented by, or incorporated in, one or more application-specific integrated circuits (ASICs) and/or one or more field programmable gate arrays (FPGAs).

Data storage device 1112 and memory 1110 each include a tangible non-transitory computer readable storage medium. Data storage device 1112, and memory 1110, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 1108 may include peripherals, such as a printer, scanner, display screen, etc. For example, input/output devices 1108 may include a display device such as a cathode ray tube (CRT) or liquid crystal display (LCD) monitor for displaying information to the user, a keyboard, and a pointing device such as a mouse or a trackball by which the user can provide input to computer 1102.

An image acquisition device 1114 can be connected to the computer 1102 to input image data (e.g., medical images) to the computer 1102. It is possible to implement the image acquisition device 1114 and the computer 1102 as one device. It is also possible that the image acquisition device 1114 and the computer 1102 communicate wirelessly through a network. In a possible embodiment, the computer 1102 can be located remotely with respect to the image acquisition device 1114.

Any or all of the systems and apparatus discussed herein, including the systems and apparatuses used to implement the random forest classifier and the logistic regression classifier utilized at step 106 of Figure 1 or the classifier utilized at step 408 of Figure 4, may be implemented using one or more computers such as computer 1102.

One skilled in the art will recognize that an implementation of an actual computer or computer system may have other structures and may contain other components as well, and that Figure 11 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention.

Those skilled in the art could implement various other feature combinations without departing from the scope of the invention.

**Claims**

1.  A computer implemented method, comprising:

    receiving (102) input medical images;
    computing (104) a plurality of metrics for a disease for each of the input medical images; and
    clustering (106) the input medical images into a plurality of clusters based on one or more of the plurality of metrics to classify the input medical images, the plurality of clusters comprising:

    a cluster of one or more of the input medical images associated with the disease, and
    one or more clusters of one or more of the input medical images not associated with the disease.

2.  The computer implemented method of claim 1, wherein clustering the input medical images into a plurality of clusters based on one or more of the plurality of metrics to classify the input medical images comprises:
    performing unsupervised hierarchical clustering based on a distance between each pair of images in the input medical images.

3.  The computer implemented method of claim 2, further comprising computing the distance between each pair of images in the input medical images by:

    computing an initial distance between same metrics of the one or more of the plurality of metrics for each respective pair of images; and
    averaging the initial distances between the same metrics for each respective pair of images.

4.  The computer implemented method of claim 1, wherein clustering the input medical images into a plurality of clusters based on one or more of the plurality of metrics to classify the input medical images comprises:
    performing a supervised classification using a random forest classifier and a logistic regression classifier.

5.  The computer implemented method of any one of claims 1 - 4, further comprising:
    selecting the one or more of the plurality of metrics that most discriminate medical images associated with the disease from medical images not associated with the disease.

6.  The computer implemented method of any one of claims 1 - 5, wherein the plurality of metrics for the disease represent a distribution, location, and extent of the disease.

7.  The computer implemented method of any one of claims 1 - 6, wherein the disease is COVID-19 (coronavirus disease 2019).

8.  A computer implemented method comprising:

    receiving (402) an input medical image of lungs of a patient;
    segmenting (404) the lungs from the input medical image;
    generating (406) a probability map for abnormality patterns associated with a disease from the input medical image; and
    determining (408) a classification of the input medical image based on the segmented lungs and the probability map, the classification representing whether the input medical image is associated with the disease.

9.  The computer implemented method of claim 8, wherein the disease is COVID-19 (coronavirus disease 2019) and the abnormality patterns associated with COVID-19 comprise opacities of one or more of ground glass opacities (GGO), consolidation, and crazy-paving pattern.

10. The computer implemented method of claim 8 or 9, wherein the classification of the input medical image is an indication that the input medical image is associated with the disease or an indication that the input medical image is not associated with the disease.

**11.** The computer implemented method of any one of claims 1 - 10, further comprising at least one the steps of:

generating the input medical images using an image acquisition device, in particular a CT or x-ray scanner or MRI (magnetic resonance imaging) device;
displaying the clustered images and/or probability map on a display device of a computer system;
storing the classification of the input medical images on a memory or storage of a computer system;
transmitting the classification of the input medical images to a remote computer system; and/or outputting the classification of the input medical images in a computer-readable form.

**12.** The computer implemented method of any one of claims 1 - 11, further comprising: using a trained machine learning based network (800) for clustering and/or classify the input medical images.

**13.** An apparatus comprising means for carrying out the steps of any one of claims 1 - 12, in particular comprising:

means for receiving (102, 402) an input medical image of lungs of a patient;
means for computing (104);
means for clustering (106);
means for performing classification;
means for segmenting (404);
means for generating (406); and/or
means for determining (408).

**14.** The apparatus of claim 13, further comprising:

an image acquisition device for generating the input medical images, in particular, a CT or x-ray scanner or MRI (magnetic resonance imaging) device; and/or
a machine learning based network (800) device implemented to perform the classification and/or clustering.

**15.** A non-transitory computer readable medium storing computer program instructions, the computer program instructions when executed by a processor cause the processor to perform operations comprising the steps of any one of claims 1 - 12.

<u>100</u>

```
┌─────────────────────────────────────────────────────────────┐
│               Receive input medical images                  │
│                          102                                │
└─────────────────────────────────────────────────────────────┘
                              │
                              V
┌─────────────────────────────────────────────────────────────┐
│  Compute a plurality of metrics for a disease for each of    │
│              the input medical images                        │
│                          104                                │
└─────────────────────────────────────────────────────────────┘
                              │
                              V
┌─────────────────────────────────────────────────────────────┐
│  Cluster the input medical images into a plurality of        │
│  clusters based on one or more of the plurality of metrics   │
│           to classify the input medical images               │
│                          106                                │
└─────────────────────────────────────────────────────────────┘
                              │
                              V
┌─────────────────────────────────────────────────────────────┐
│    Output the classification of the input medical images     │
│                          108                                │
└─────────────────────────────────────────────────────────────┘
```

Figure 1

200

Figure 2A

210

Figure 2B

300

Figure 3

400

```
┌─────────────────────────────────────────────────────────┐
│         Receive an input medical image of lungs of a patient      │
│                             402                           │
└─────────────────────────────────────────────────────────┘
                              │
                              ▼
┌─────────────────────────────────────────────────────────┐
│          Segment the lungs from the input medical image          │
│                             404                           │
└─────────────────────────────────────────────────────────┘
                              │
                              ▼
┌─────────────────────────────────────────────────────────┐
│   Generate a probability map for abnormality patterns associated with a disease   │
│                    from the input medical image                   │
│                             406                           │
└─────────────────────────────────────────────────────────┘
                              │
                              ▼
┌─────────────────────────────────────────────────────────┐
│  Determine a classification of the input medical image based on the segmented   │
│  lungs and the probability map, the classification representing whether the input  │
│             medical image is associated with the disease          │
│                             408                           │
└─────────────────────────────────────────────────────────┘
                              │
                              ▼
┌─────────────────────────────────────────────────────────┐
│          Output the classification of the input medical image     │
│                             410                           │
└─────────────────────────────────────────────────────────┘
```

Figure 4

500

| 2 classes | 4 categories | Train | Validation | Test |
|-----------|--------------|-------|------------|------|
| Positive | COVID-19 | 1000 | 50 | 100 |
| Negative | Pneumonia | 113 | 16 | 30 |
| | ILD | 131 | 16 | 30 |
| | Without pathology | 559 | 17 | 34 |

# Figure 5

Figure 6

Figure 7

<u>800</u>

| | | Ground Truth | | | |
|---|---|---|---|---|---|
| | | Positive | Negative | | |
| | | COVID-19 | ILD | Pneumonia | Healthy |
| Predicted (M1) | Positive | 74 | 13 | 12 | 0 |
| | Negative | 26 | 17 | 18 | 34 |
| Predicted (M2) | Positive | 81 | 12 | 10 | 0 |
| | Negative | 19 | 18 | 20 | 34 |
| Predicted (M3) | Positive | 86 | 6 | 12 | 0 |
| | Negative | 14 | 24 | 18 | 34 |

Figure 8

Figure 9

Figure 10

Figure 11

Europäisches
Patentamt

European
Patent Office

Office européen
des brevets

# EUROPEAN SEARCH REPORT

Application Number

EP 21 18 0032

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Georgescu Bogdan ET AL: "Machine Learning Automatically Detects COVID-19 using Chest CTs in a Large Multicenter Cohort Authors", arXiv e-prints, 11 June 2020 (2020-06-11), XP055858989, Retrieved from the Internet: URL:https://arxiv.org/vc/arxiv/papers/2006/2006.04998v2.pdf [retrieved on 2021-11-08] * the whole document, in particular the abstract, pages 7-9, and figures 1, 2a, 2b,3, 4 and 5 * | 1-15 | INV. G16H30/40 G16H50/20 G16H50/80 |
| X | FANG MENGJIE ET AL: "CT radiomics can help screen the Coronavirus disease 2019 (COVID-19): a preliminary study", SCIENCE CHINA INFORMATION SCIENCES, SCIENCE CHINA PRESS, BEIJING, vol. 63, no. 7, 15 April 2020 (2020-04-15), XP037095410, ISSN: 1674-733X, DOI: 10.1007/S11432-020-2849-3 [retrieved on 2020-04-15] * the whole document, in particular the abstract, section "2 Methods" and section "3 Results" on pages 2-6 * | 1-15 | |

TECHNICAL FIELDS SEARCHED (IPC)

G16H

The present search report has been drawn up for all claims

| Place of search | Date of completion of the search | Examiner |
|---|---|---|
| Munich | 10 November 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 83797920